# EUROPEAN PATENT APPLICATION

(11) **EP 2 711 367 A1**
(43) Date of publication of application: **26.03.2014**
(21) Application number: 12006666.7
(22) Date of filing: 22.09.2012
(51) Int. Cl.: C07D 491/107, A61K 31/4188, A61K 31/4192, A61P 35/00

(54) **New spiroepoxide tetrahydrobenzo-triazoles and -imidazoles and their use as MetAP-II inhibitors**

(71) Applicant: Deutsches Krebsforschungszentrum, 69120 Heidelberg (DE); Universität Heidelberg, 69117 Heidelberg (DE)
(72) Inventor: Miller, Aubrey, 69115 Heidelberg (DE); Jöst, Christiaan, 69191 Heidelberg (DE); Klein, Christian, 69214 Eppelheim (DE)
(74) Representative: Schüssler, Andrea

(57) **Abstract**

The present invention relates to new spiroepoxide tetrahydrobenzo-triazole and - imidazole compounds, a method for their production and their use as MetAP-II inhibitors, which are particularly useful as inhibitors of angiogenesis. The compounds are characterized by formula (I) 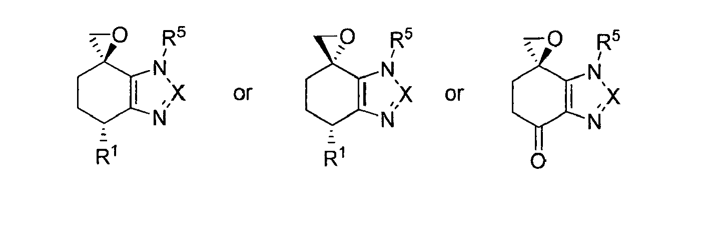 X=CorN
R¹ = OC(O)R² where R² is alkyl, cycloalkyl, aryl, heteroaryl, or cinnamyl or
R¹ = OC(O)NHR³ where R³ is alkyl, cycloalkyl, aryl, or heteroaryl
or
R¹ = NHC(O)OR⁴ where R⁴ is alkyl
or
R¹ = OH, NH₂
R⁵ = alkyl, cycloalkyl, CH₂R⁶ where R⁶ is aryl or heteroaryl

## Description

### BACKGROUND OF THE INVENTION

The present invention relates to new spiroepoxide tetrahydrobenzo-triazole and imidazole compounds, a method to their production and their use as MetAP-II inhibitors, which are particularly useful as inhibitors of angiogenesis.

Angiogenesis, the growth of new blood capillaries from pre-existing ones is a vital process in many physiological processes, including embryonic development, growth, and wound-healing. Since Folkman's discovery (W. D. Figg, J. Folkman, Angiogenesis: an integrative approach from science to medicine, 1st ed., Springer, New York, 2008; J. Folkman, N. Engl. J. Med. 1971, 285, 1182-1186; J. Folkman, Nat. Rev. Drug Discov. 2007, 6, 273-286) that angiogenesis is linked to pathological conditions (most famously cancer but also non-neoplastic diseases) much effort has been devoted to the development of anti-angiogenic based therapies. Indeed, a number of small molecule and antibody-based anti-angiogenics are now FDA approved for the treatment of cancer.

For example, the inhibition of the tumor-associated angiogenesis, i.e. directed against the formation of new blood vessels, is a new option for treating solid tumors and haematological malignancies (Moehler, M. et al., Curr. Pharm. Des., 2004: 11, 1221-1234; Ribatti, D. et al., Stem Cell Development, 2004: 13, 484-495). An effective agent against tumor angiogenesis should fulfil multiple criteria, such as: a) efficiency against different types of tumors, b) small risk of resistance, c) efficiency against different mechanisms of tumor-associated angiogenesis, d) low toxicity, and e) possible combination with other forms of therapies, e.g. chemotherapy. However, most of the already existing active agents do not meet these criteria.

Up to now, the previously used or tested active agents for the inhibition of the tumor-associated angiogenesis are predominantly based on the protein portion of specific angiogenic factors or receptors. For example, since the vascular endothelial growth factor (VEGF) stimulates the growth of new blood vessels, anti-VEGF therapies are important in the treatment of certain tumors. These treatments involve monoclonal antibodies such as bevacizumab (tradename "Avastin"), which show therapeutic efficiency in mouse tumor models and in an increasing number of human tumors. Furthermore, drugs against the corresponding VEGF-receptor, e.g. semaxanib (SU5416), vandetanib (ZD6474) or CP-547,632 (Malik, A. K. and H.-P. Gerber, Targets, 2003: 2, 48-57) and drugs with a wider spectrum, e.g. thalidomide, interferon, such as IFN-α or IFN-β, celecoxib, ZD6126 and fumagillin are in use or rather currently part of clinical studies as anti-angiogenic agents. Further drugs, such as lanildomide (tradename "Revlimid"), a derivative of thalidomide, induce tumor cell apoptosis directly and indirectly, e.g. by anti-angiogenic effects.

Tumor angiogenesis is the proliferation of a network of blood vessels that penetrates into cancerous tissue, supplying nutrients and oxygen to the tumor and removing waste products from the tumor. Tumor angiogenesis actually starts with cancerous tumor cells releasing molecules that send signals to surrounding normal host tissue. This signaling activates certain genes in the host tissue that, in turn, make proteins to encourage growth of new blood vessels.

More specifically, tumor angiogenesis comprises several overlapping phases where the endothelial cells (EC) are involved: (1) activation of the endothelial cells, i.e. biological signals known as angiogenic growth factors activate receptors on endothelial cells present in pre-existing blood vessels; (2) adhesion and migration of the endothelial cells and formation of a provisional blood vessel, i.e. the activated endothelial cells begin to release enzymes called proteases that degrade the basement membrane to allow endothelial cells to escape from the original (parent) vessel walls. The endothelial cells then proliferate into the surrounding matrix and form solid sprouts connecting neighbouring vessels. As sprouts extend towards the source of the angiogenic stimulus, endothelial cells migrate in tandem, using adhesion molecules, called integrins; (3) stabilisation of the new blood vessel by perivascular cells, e.g. fibroblasts and pericytes, i.e. the sprouts then form loops to become a full-fledged vessel lumen as cells migrate to the site of angiogenesis. Sprouting occurs at a rate of several millimetres per day and enables new vessels to grow across gaps in the vasculature. The tumor vessels show several differences to normal blood vessels and capillaries, i.e. incomplete and irregularly formed basal membranes and large interendothelial gaps.

Anti-angiogenic drugs which are intended for treating solid tumors as well as haematological malignancies lead to a certain improvement of the progression-free phase and the median overall survival of the patients, also in combination with chemotherapy. However, these drugs cannot prevent the overall progression of the malignant diseases and do not have a curative effect. This is mostly due to the fact that tumor cells can develop a certain resistance against this anti-angiogenic treatment, e.g. by using mechanisms of vascularization other than VEGF-induced angiogenesis. Furthermore, for some (unknown) reason some patients remain irresponsive to this therapy. Moreover, some therapeutics are associated with severe side effects, e.g. disorders of the peripheral or central nervous system, disorders of the gastrointestinal-tract or slowed heartbeat (bradycardia) und heart failure.

Thus, the development of further angiogenesis inhibitors with an independent mechanism of action is necessary. Methionine aminopeptidase enzymes (MetAPs) are metalloproteases that are responsible for the co-translational cleavage of initiator methionines from ribosomally synthesized proteins. This process is essential for cell function and, therefore, pharmacological disruption of this metabolic step can lead to cell death. In higher eukaryotic organisms, the MetAP functionality is redundant with two subtypes of the enzyme. Selective inhibition of the MetAP-II subtype is not lethal, but stops the growth of endothelial cells in culture, inhibits angiogenesis in animal models, and is a well validated strategy for anti-angiogenic cancer therapy. While a number of reversible small molecule MetAP-II inhibitors have been described, the highly potent natural products fumagillin (2), ovalicin (7), and their derivatives are arguably the most well-known and well-studied MetAP-II inhibitors (Scheme 1a). TNP-470 (3) was entered into Phase II clinical trials, but has an extremely short plasma half-life (~2 min) and exhibited CNS-related side effects. PPI-2458 (4) has been reported to have a longer plasma half-life and no CNS effects while ZGN-433 (5) should enter Phase II clinical trials within the year.

Fumagillin (2) irreversibly inhibit MetAP-II by forming a covalent bond between the spiro-epoxide C2 and a histidine residue (H231) in the active site of the enzyme (Scheme 1b)

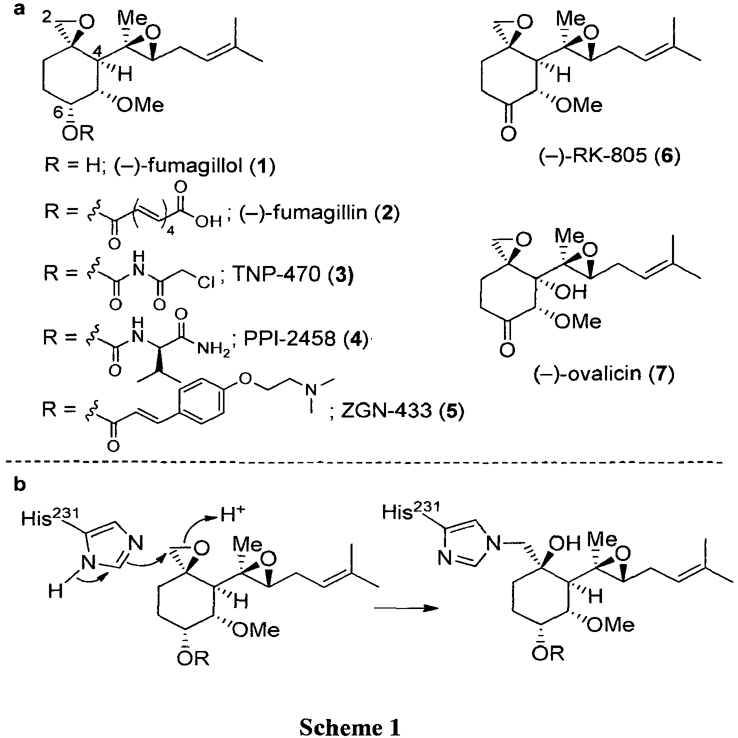

Although the use of anti-angiogenic compounds has particularly been tested in connection with cancer therapy, it is advantageous to inhibit angiogenesis also for treating ophthalmic diseases, skin diseases, rheumatoid arthritis and obesity.

Thus, the problem of the present invention is the provision of further MetAP-II inhibitors which show a better biological activity than known MetAP-II inhibitors, enhanced inhibitory potential and selectivity for the desired MetAP-subtype and do not have the known side effects of known fumagillin derivatives.

The problem is solved by the subject-matter of the present claims 1, 4, 6, 9 and 10. Preferred embodiments are the subject-matter of the dependent claims.

### SUMMARY OF THE INVENTION

The present invention relates to spiroepoxide tetrahydrobenzo-triazole and -imidazole compounds which show a biological activity at low concentrations similar to known fumagillin derivatives in inhibiting angiogenesis based on inhibiting the enzymatic activity of the enzyme MetAP-II. They would address some of the pharmacological liabilities, i.e. high lipophilicity, low-solubility, short half-life and rapid clearance. The present invention further relates to pharmaceutical compositions comprising these new compounds. The present invention also relates to a method for preparing the epoxytriazole and epoxyimidazole compounds of the present invention.

### DETAILED DESCRIPTION OF THE INVENTION

The present invention relates to epoxytriazole and epoxyimidazole compounds of the claimed Formula (I) showing a biological activity comparable to known MetAP-II inhibitors for inhibiting angiogenesis. The inhibition of angiogenesis is particularly useful for treating cancer and inhibiting the formation of metastasis by inhibiting adhesion and/or migration of cancer cells.

Thus, the present invention concerns compounds that are represented by the general Formula (I): 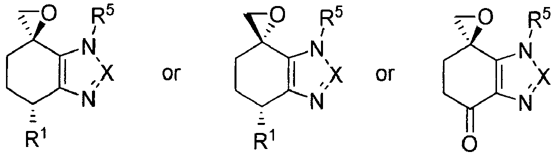 x = C or N
R¹ = OC(O)R² where R² is alkyl, cycloalkyl, aryl, heteroaryl, or cinnamyl
or
R¹ = OC(O)NHR³ where R³ is alkyl, cycloalkyl, aryl, or heteroaryl
or
R¹ = NHC(O)OR⁴ where R⁴ is alkyl
or
R¹ = OH, NH₂
R⁵ = alkyl, cycloalkyl, CH₂R⁶ where R⁶ is aryl or heteroaryl

If not stated otherwise, in the present invention the "alkyl" residue (preferably: C₁ to C₁₀) can be linear or branched, unsubstituted or substituted. Preferred alkyl residues are methyl, ethyl, n-propyl, iso-propyl, n-butyl, tert-butyl, n-pentanyl, n-hexanyl. The term "alkyl", also in word combinations such as alkylsulfonyl, alkylamino or alkoxycarbonyl *etc.,* mean both unbranched and branched possible compounds. The same also applies to the corresponding cycloalkyl compounds having preferably 3 to 10 carbon atoms. "Aryl" refers to an aromatic monocyclic or polycyclic ring system having 6 to 14 carbon atoms, preferably 6 to 10 carbon atoms. The aryl group can be substituted, where appropriate, with one or several ring substituents. Preferred aryl groups are phenyl or naphthyl.

"Heteroaryl" (sometimes referred to as "hetaryl") denotes an aromatic monocyclic or polycyclic ring system having 5 to 14 ring atoms, preferably 5 to 10 ring atoms, where 1 or more ring atoms are an element other than carbon, e.g. N, O or S, as such or in combination. Preferred heteroaryls contain 5 or 6 ring atoms. The heteroaryls may be substituted, where appropriate, at one or more ring systems. Examples of suitable heteroaryls are: pyridyl, pyrazinyl, pyridinyl, furanyl, thienyl, pyrimidinyl, isoxazolyl, isothiazolyl, oxazolyl, thiazolyl, pyrazolyl, furazanyl, pyrrolyl, triazolyl, 1,2,4-thiadiazolyl, pyrazinyl, pyridazinyl, benzofurazanyl, indolyl, azaindolyl, benzoimidazolyl, benzothienyl, quinolinyl, imidazolyl, thienopyridinyl, 1,2,4-triazinyl, benzothiazolyl or benzoazaindolyl.

"Cinnamyl" means a 3-phenylprop-2-enyl group (-> H₅C₆-CH=CH-CH₂-).

As mentioned before, the alkyl, cycloalkyl, aryl, hetaryl and cinnamyl compounds may be further substituted, where appropriate, with the following substituents:
-OH, -SH, -O-C₁₋₈ alkyl, -O-C₆₋₁₄ aryl, -S-C₁₋₄ alkyl, -S-C₆₋₁₄ aryl, -SO-C₁₋₄ alkyl, -SO-C₆₋₁₄ aryl, -SO₂-C₁₋₄ alkyl, -SO₂-C₆₋₁₄ aryl, -SO₃H, -OSO₂C₁₋₈ alkyl, -OSO₂C₆₋₁₄ aryl, -COOH, -CONH₂, -CONHC₁₋₆ alkyl, -CON(C₁₋₆ alkyl)₂, -NH₂, -NHC₁₋₆ alkyl, -N(C₁₋₆ alkyl)₂, -NHC₆-₁₄ aryl, -NH-hetaryl, -C₁₋₆ alkyl, -C₂₋₁₂ alkenyl, -C₂₋₁₂ alkynyl, each straight-chain, branched or cyclic as well as substituted, where appropriate, once, twice or thrice with halogen independently of one another, -halogen (-F, -Cl, -Br, -I) -CH₂CH₂OH, -CH₂CH₂SH, -CH₂CH₂SCH₃, -amidino, hydroxyamidino -sulfo, phosphono, - -CN, -NO₂ and -SCN

In the sense of the invention, all residues are considered combinable with one another unless stated otherwise in the definition of the residues. All conceivable subgroups thereof shall be considered disclosed.

The invention also relates to physiologically compatible salts of the compounds of general formula (1). The physiologically compatible salts are obtained as usual by reaction of basic compounds of general formula (1) with inorganic or organic acids, where appropriate, also in the presence of compounds having acidic properties. Hydrochloric acid, sulphuric acid, nitric acid or hydrobromic acid are preferably used as inorganic acids and e.g. formic acid, acetic acid, propionic acid, glycolic acid, lactic acid, amygdalic acid, tartaric acid, malic acid, citric acid, malonic acid, maleic acid, fumaric acid, succinic acid, alginic acid, benzoic acid, 2-, 3- and 4-alkyloxy and acyloxy benzoic acids, ascorbic acid, C₁-C₃ alkylsulfonic acids, benzenesulfonic acid, nicotinic acid, isonicotinic acid and amino acids are used as organic acids. For example, ammonia, soda lye and caustic potash solution are used as inorganic bases and alkylamines, C₁-C₃ pyridine, quinoline, isoquinoline, piperazine and derivatives thereof, and picolines, quinaldine or pyrimidine are used as organic bases.

The invention also relates to solvates of the compounds, including the pharmaceutically acceptable salts, acids, bases and esters as well as the active metabolites thereof and, where appropriate, the tautomers thereof according to general formula (1) including prodrug formulations. Prodrug formulations here comprise all substances which are formed by simple transformation including hydrolysis, oxidation or reduction either enzymatically, metabolically or in any other way. A suitable prodrug contains e.g. a substance of general formula (1) bound via an enzymatically cleavable linker (e.g. carbamate, phosphate, N-glycoside or a disulfide group) to a dissolution-improving substance (e.g. tetraethylene glycol, saccharides, formic acids or glucuronic acid, *etc.).* Such a prodrug of a compound according to the invention can be applied to a patient, and this prodrug can be transformed into a substance of general formula (1) so as to obtain the desired pharmacological effect.

Some compounds of Formula (I) are encompassed in form of the racemates, their enantiomers and optionally in form of their diastereomers and all possible mixtures thereof.

The obtained compounds can be optionally separated by known methods (e.g. Allinger, N.L. und Elliel E.L. in "Topics in Stereochemistry "Vol. 6, Wiley Interscience, 1971*)* in their enantiomers and/or diasteromers. One possible method of enantiomeric separation is the use of chromatography.

The compounds according to the invention can be administered in different ways, e.g. orally, parenterally, cutaneously, subcutaneously, intravenously, intramuscularly, rectally, or by inhalation. The intravenous administration or administration by inhalation is preferred. The compound is given to a patient who needs a therapy for a disease coming under the indication spectrum of the compounds according to the invention over a period to be determined by a physician. The compound can be administered to both humans and other mammals.

The dosage of the compounds according to the invention is determined by the physician on the basis of the patient-specific parameters, such as age, weight, sex, severity of the disease, *etc.* The dosage is preferably from 0.001 mg/kg to 1000 mg/kg body weight, preferably from 0.01 to 500 mg/kg body weight and most preferably from 0.1 to 100 mg/kg body weight.

Corresponding to the kind of administration, the medicament is suitably formulated, e.g. in the form of solutions or suspensions, simple tablets or dragees, hard or soft gelatine capsules, suppositories, ovules, preparations for injection, which are prepared according to common galenic methods.

The compounds according to the invention can be formulated, where appropriate, together with further active substances and with excipients common in pharmaceutical compositions, e.g. - depending on the preparation to be produced - talcum, gum arabic, lactose, starch, magnesium stearate, cocoa butter, aqueous and non-aqueous carriers, fatty bodies of animal or vegetable origin, paraffin derivatives, glycols (in particular polyethylene glycol), various plasticizers, dispersants or emulsifiers, pharmaceutically compatible gases (e.g. air, oxygen, carbon dioxide, *etc.),* preservatives.

In order to produce liquid preparations, additives, such as sodium chloride solution, ethanol, sorbitol, glycerine, olive oil, almond oil, propylene glycol or ethylene glycol, can be used.

When solutions for infusion or injection are used, they are preferably aqueous solutions or suspensions, it being possible to produce them prior to use, e.g. from lyophilized preparations which contain the active substance as such or together with a carrier, such as mannitol, lactose, glucose, albuimin and the like. The ready made solutions are sterilized and, where appropriate, mixed with excipients, e.g. with preservatives, stabilizers, emulsifiers, solubilizers, buffers and/or salts for regulating the osmotic pressure. The sterilization can be obtained by sterile filtration using filters having a small pore size according to which the composition can be lyophilized, where appropriate. Small amounts of antibiotics can also be added to ensure the maintenance of sterility.

Furthermore, inhalation compositions, e.g. in the form of aerosols, sprays or as micronized powder, are preferably produced. For this purpose, the compounds according to the invention are either dissolved or suspended in pharmaceutically conventional solvents and finely divided by means of excess pressure in a certain volume and inhaled. The procedure is made correspondingly in the solid substances to be inhaled which are also finely divided by means of excess pressure and inhaled. Other applicators working by means of excess pressure are also included here.

The invention also relates to pharmaceutical preparations which contain a therapeutically active amount of the active ingredients (compound according to the invention of formula (1)) together with organic or inorganic solid or liquid, pharmaceutically compatible carriers which are suited for the intended administration and which interact with the active ingredients without drawbacks:
Within the meaning of the invention, all residues are considered combinable unless stated otherwise in the definition of the residues. All conceivable subgroupings thereof are considered to be disclosed.

Preferred compounds of the present invention are e.g.

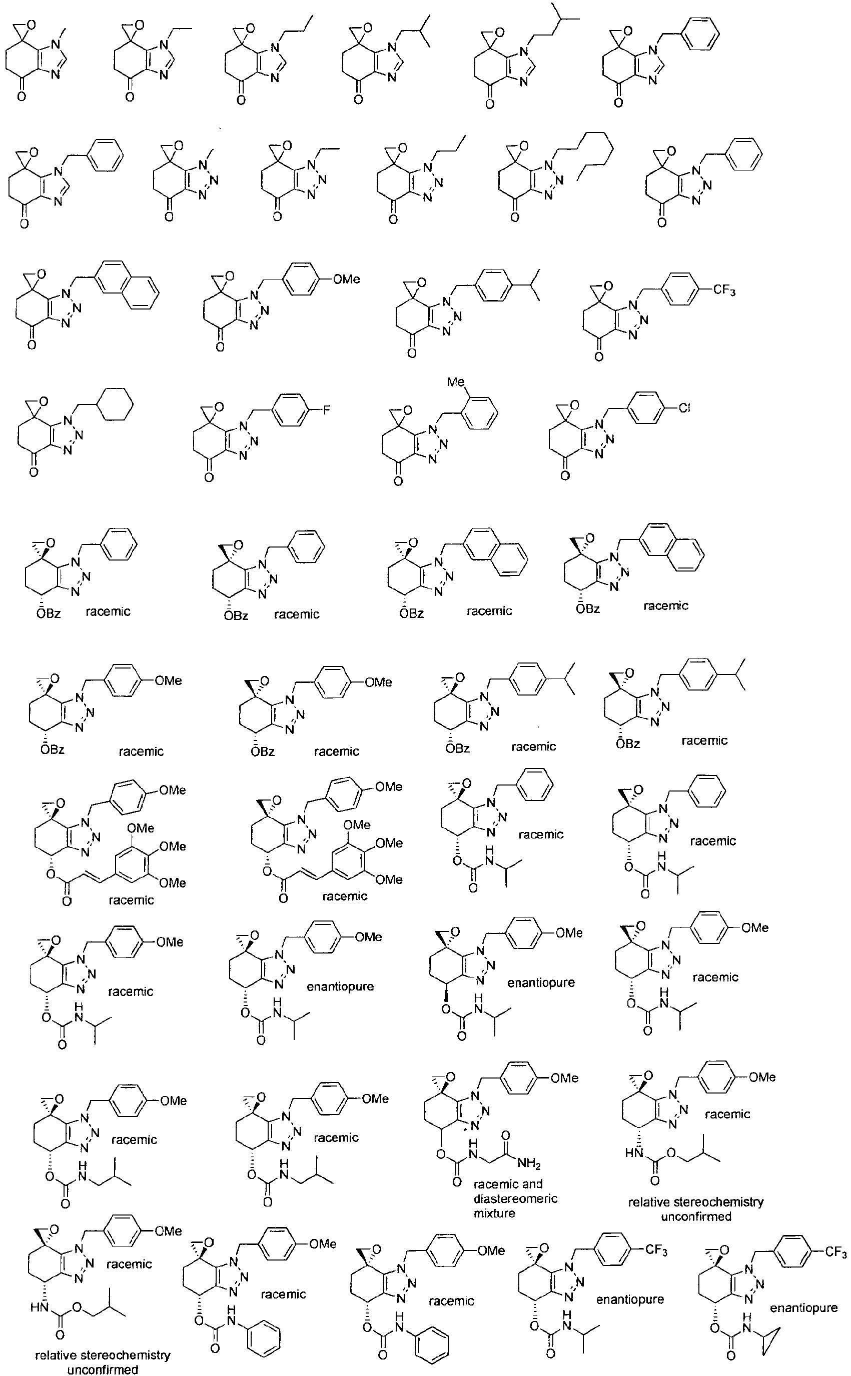

The synthesis of the compounds of the present invention is described in detail in the example section. An overview of the triazole synthesis is given in Scheme 2. The optimized synthetic route began with a one-pot reaction: iodination and subsequent deprotonation of ethynylmagnesium bromide (**8**) gave lithium iodoacetylide which reacted with Weinreb amide **9** to give iodoynone **10**, a compound prone to decomposition. In spite of the sensitive nature of this compound, with the described method, it was possible to reproducibly perform this reaction on 50 mmol scale. Alkyne **10** served as a reacting partner with a variety of azides in copper catalyzed Huisgen cyclizations to generate triazoles **11.** After optimization, the inventors found that Jeffery's phase transfer method under rigorously degassed conditions gave reliable yields of bicyclic olefins **12**. Epoxidation of **12** with DMDO cleanly provided RK-805 (**6**) analogs **13** while DIBAL reduction of **12** gave alcohols **14.** These alcohols could be esterified and subsequently oxidized with DMDO to give a 1:1 diastereomeric mixture of epoxides **16** and **17**. The inventors were unable to assign the relative stereochemistry of the two compounds using NMR, but crystallographic analysis of single crystals grown from one example of **16** revealed this diastereomer to have the fumagillin-like "trans" relationship of the oxirane and carboxylate oxygens at C3 and C6, respectively. All similarly generated sets of epoxide diastereomers (vide infra) were subsequently assigned on the basis of distinctive differences in the ¹H NMR spectra of **16** and **17** and predictable relative R_{f} values. Alcohols **14** could also be converted to the activated carbonates **18** and subsequently transformed into carbamates **19**. Epoxidation with DMDO again gave a set of separable diastereomeric products **20** and **21**. Ketones **12** underwent reductive amination to give amines **22**, which could be converted into carbamates **23** and epoxides **24** and **25**.

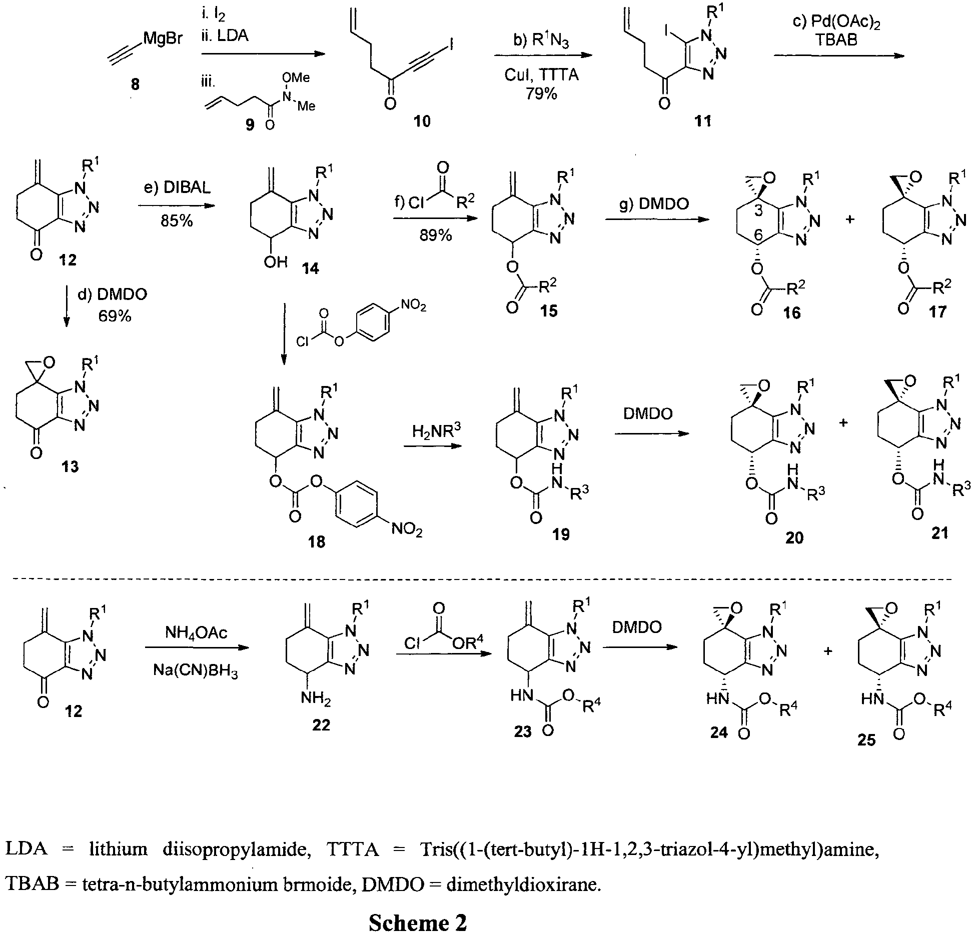

An overview of the imidazole synthesis is shown in Scheme 3. Magnesium/halogen exchange on diiodoimidazole **(26)** using Knochel's procedure, followed by addition of aldehyde **27,** gave alcohol **28** as a crystalline racemic solid. Oxidation with IBX gave ketone **29** which could be alkylated with a variety of electrophiles to give imidazoles **30.** Heck cyclization as above gave the bicyclic olefins **31** which could be epoxidized with DMDO to give the final products **32.**

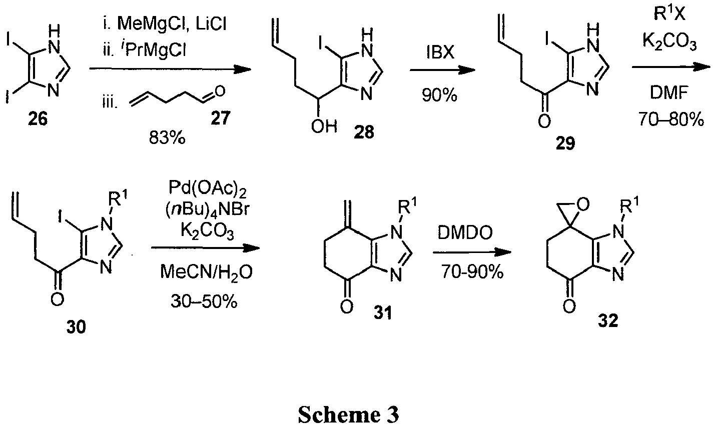

The inventors also wanted to determine the more active enantiomer of a potent substance, carbamate **39** (c.f. Scheme 4). Chiral chromatographic separation of (±)-**39** provided (+)-**39** and (-)-**39**. The absolute stereochemistry of the two enantiomers was determined through comparison of their chiral HPLC retention times with a sample of enantioenriched (+)-**39** which was synthesized as shown in Scheme 4. Starting with known protected alcohol (+)-**33** (prepared by Noyori reduction of the corresponding TMS-protected ynone and subsequent protecting group manipulations), iodination gave alkyne **34**. This compound smoothly underwent copper catalysed Huisgen cyclization to give triazole **35** which was then subjected to Heck cyclization and silyl deprotection to give alcohol (*R*)-**37**. Two step conversion of **37** to carbamate **38** followed by epoxidation gave (+)-**39** (>95% ee). Biochemical testing of the two enantiomers showed that (+)-**39**, having the same absolute stereochemistry as fumagillin, was significantly more active against hsMetAP-II.

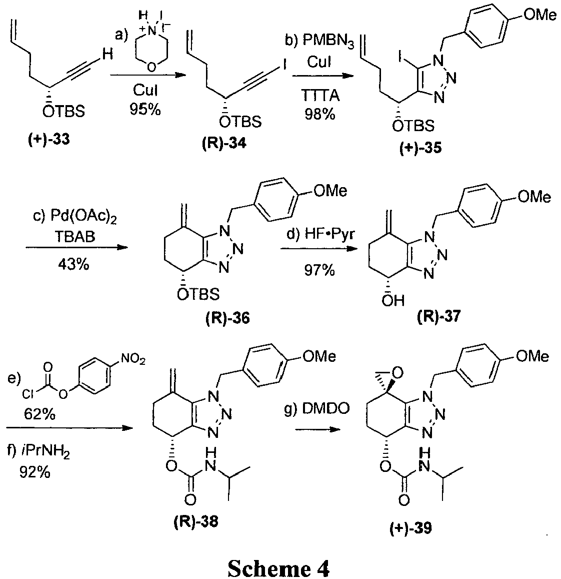

The inventors prepared a series of triazoles and imidazoles in analogy to the above described schemes and measured their inhibitory activity against three different enzymes (*E. coli* MetAP, hsMetAP-I, and hsMetAP-II) in a biochemical screen at a single concentration. All compounds were found to be essentially inactive against both *E*. *coli* MetAP and hsMetAP-I while many were potent inhibitors of hsMetAP-II. IC₅₀ values against hsMetAP-II were then measured for compounds with reasonable potency. The alkyl side-chain derivatives were active against hsMetAP-II but some substituted benzyl derivatives showed much more promising activity, exhibiting sub-micromolar IC₅₀ values. The most promising substances are shown in Scheme 5 below. Importantly, all non-epoxide intermediates were tested and found to be completely inactive against hsMetAP-II.

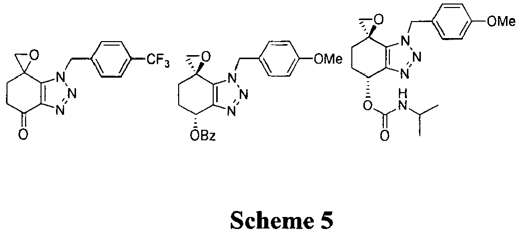

Selected substances were tested for their ability to arrest the growth of human umbilical vein endothelial cells (HUVEC), a hallmark of MetAP-II inhibitors (c.f. Table 1). With reference to Table 1 the compounds HHS-I-028-B, BK-I-161-A, UWU-XII-023-A, UWU-XII-035-A, UWU-XII-128-A, and (+)-UWU-XII-131-A show a good inhibitory effect on MetAP-II and are thus, useful as anti-angiogenic compounds. While some compounds, particularly non-epoxide substances showed general toxicity at high concentrations, many of the epoxides exhibited biphasic inhibition, i.e. within a certain compound concentration range the HUVECs did not die but stopped dividing, while at concentrations above this range, the HUVECs were killed. Relatively good correlation can be observed between hsMetAP-II and HUVEC growth inhibition IC₅₀ values. In particular, the following compound showed excellent potency in both assays.

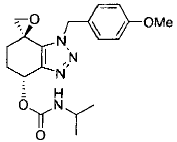

In conclusion the inventors provide the synthesis and preliminary biological evaluation of a new chemical class of spiroepoxide tetrahydrobenzotriazoles, inspired by the natural product fumagillin (**2**), that show potent and selective inhibition of MetAP-II. The inventors have provided evidence that these substances function as irreversible inhibitors and that they show antiproliferative activity against HUVECs.

With the performed experiments it was confirmed that
1. The compounds of the present invention have an inhibitory influence on the endothelial cell mediated angiogenesis already at an early stage.
2. The compounds of the present invention do not have a negative influence on the vitality and proliferating ability of the endothelial cells
3. In view of the inhibitory effect on the adhesion also later phases of the angiogenesis will be inhibited. This means that the whole process of building new vessels will be inhibited. Thus, the compounds of the present invention are perfect anti-angiogenic compounds.

The invention also relates to pharmaceutical compositions containing a therapeutically active amount of one or more (= at least one) of the compounds of the present invention. The compounds of the present invention are therapeutically particularly useful for those diseases where cell migration and neoangiogenesis plays a role. Thus, the treatment of all types of neoplasms and tumors, whether benign or malign, would be possible by the administration of the compounds. Neoplasms may be malignant, e.g. carcinoma, sarcoma, glioblastoma, astrocytoma, neuroblastoma, retinoblastoma, etc. The terms "malignant neoplasm" and "malignant tumor" refer to a neoplasm that contains at least one cancer cell. Neoplastic cells are typically invasive, i.e. they invade adjacent tissues by cell migration or are shed from the primary site and circulate through the blood and lymph to other locations in the body where they initiate one or more secondary cancers (metastases). Thus, the term "cancer" is used herein to refer to a malignant neoplasm, which may or may not be metastatic. In particular, the compounds of the present invention are useful for treating any cancers located in human or animal organs, brain, skin or hematological neoplasia. Examples are melanomas, basal cells carcinoma, Kaposi sarcoma, glioma, cancer of the lung, bladder, prostate, kidney, testis, intestine, colon, spleen, cervix, ovary, endometrium, uterus, esophagus, tongue, bronchi, pancreas, liver, breast, stomach or bone, leukemia (ALL, CLL, AML), lymphoma, myeloma (multiple myelomas, Hodgkin-, non-Hodgkin-). Furthermore, the compounds of the invention are also useful for treating diseases, which are generally involved by neoangiogenesis, such as ophthalmic diseases (macular degeneration, diabetic retinopathy), skin diseases (psoriasis, hemangiomas), rheumatoid arthritis and obesity.

In addition, the compounds of the present invention may be useful as diagnostic tools in any imaging process. For this utility they may carry tags or labels, e.g. fluorescence, radioactivity, ³H, ¹⁴C, ¹⁸F, Iodine. The imaging processes may be computer tomography (CT), magnetic resonance tomography (MRT) or positron emission tomography (PET).

The compounds according to the invention can be administered in various ways, e.g. orally, parenterally, cutaneously, subcutaneously, intravenously, intramuscularly, rectally or by inhalation. The oral in acid resistant formulations (capsules) or inhalational administration is preferred. The compound is given to a patient who is in need of a therapy of a disease coming under the indication spectrum of the compounds according to the invention over a period to be determined by a physician. The compound can be administered to both humans and other mammals.

The dosage of the compounds according to the invention is determined by the physician by means of the patient-specific parameters, such as age, weight, sex, severity of the disease, *etc.* The dosage is preferably between 0.001 mg/kg and 1000 mg/kg body weight, more preferably 0.01 and 500 mg/kg body weight and most preferably 0.1 and 100 mg/kg body weight.

In accordance to the kind of administration, the medicament is formulated suitably, e.g. as solutions or suspensions, simple tablets or coated tablets (dragées), hard or soft gelatine capsules, powder for reconstitution prior to use, aerosols, inhalation sprays, active substance plasters, granules, suppositories, ovules, injectables, creams, ointments, gels, microspheres, implants, which are produced according to conventional galenic processes.

The compounds according to the invention can optionally be formulated with further active substances and with excipients common in pharmaceutical compositions, depending on the preparation to be produced e.g. talcum, gum arabic, lactose, starch, magnesium stearate, cacao butter, aqueous and non-aqueous carriers, adipoids of animal or vegetable origin, paraffin derivatives, glycols (in particular polyethylene glycol), various plasticizers, preservatives, dispersants or emulsifiers.

Additives, such as sodium chloride solution, ethanol, sorbitol, glycerol, olive oil, almond oil, propylene glycol, ethylene glycol or other additives common in pharmacy, can be used for the production of liquid preparations.

When infusion or injectable solutions are used, these are preferably aqueous solutions or suspensions, which can be produced prior to use, e.g. from lyophilized preparations which contain the active substance as such or together with a carrier, such as mannitol, lactose, glucose, albumin and the like. The ready-to-use solutions are sterilized and, if required, mixed with excipients, e.g. preservatives, stabilizers, emulsifiers, solubilizers, buffers and/or salts for regulating the osmotic pressure. The sterilization can be obtained by sterile filtration through filters having a small pore size whereupon the composition may be lyophilized, where necessary. Small amounts of antibiotics can also be added to maintain sterility.

The invention also relates to pharmaceutical compositions containing a therapeutically active amount of the active ingredient together with organic or inorganic solid or liquid pharmaceutically compatible carriers which are suited for the intended administration and which do not unfavourably interact with the active ingredients.

The invention also relates to processes for the production of pharmaceutical compositions, which are characterized in that the compound according to the invention is mixed with a pharmaceutically compatible carrier.

Based on combination therapies with already known active substances, the compounds according to the invention are suited for treating various diseases. Here, unexpected synergistic effects for increasing the therapeutic effectiveness of the substances according to the invention shall be utilized. On the one hand, the combination can consist of offering a single pharmaceutical composition which contains at least one of the compounds according to the invention in combination with one or more of other known active substances or the patient is given several preparations containing one or more of the known active substances simultaneously with, or time-staggered with respect to, the pharmaceutical composition according to the invention. The known active agents may be selected from the group consisting of nucleoside analogs (e.g. fludarabin, cladribin), alkylating agents (e.g. chlorambucil, cyclophosamid), corticosteroids, angiogenesis inhibitors (e.g. bevacizumab, semaxanib, vandetanib) thalidomide, interferon, and anti-cancer antibodies (e.g. Herceptin).

The invention is further described with regard to the following examples which are not construed as any limitations of the invention.

### Example 1

### General Methods

All chemical reactions were carried out under an atmosphere of argon in standard glassware. All reagents and solvents were bought from Sigma-Aldrich, Roth, VWR, Fluka and used as received, unless otherwise stated. THF was always freshly distilled from sodium prior to use. All other dry solvents were bought from Aldrich and used without further purification. Dry and/or degassed solvents or reagents including water or oxygen sensitive compounds were stored in sealed solvent bombs under Argon. 4-Pentenoic-acid was bought from SAFC and used as received. Reactions were monitored using TLC glass plates from Merck and were stained using one of the following stains: potassium permanganate, iodine p-anisaldehyde/sulphuric acid, vanillin/sulphuric acid and/or the fluorescence quenching was used for detection. Silica Gel 60 was used for flash column chromatography. MPLC was done on a "Teledyne ISCO Combiflash R_{f}" using the prepacked commercially available normal phase (SiO₂) columns in sizes as stated, always coating the crude product on coarse Silica Gel 60. Given reaction temperatures relate to the temperature of the cooling/heating baths, unless otherwise stated The cooling baths used were ice (0° C), ice/sodium chloride (-20°C), dry ice/acetone (-78°C) and liq. N₂/ H₂CCl₂ (-100° C).

The dimethyldioxirane solution was prepared and stored according to the procedure described by W. Adam et al. (Adam, W.; Bialas, J.; Hadjiarapoglou L.; Chem. Ber. 1991, 124, 1377).

NMR spectra were recorded on a "Bruker Ultrashield 400" at room temperature in deuterated Chloroform (unless otherwise stated).

For the measurement of the HUVEC growth arrest (Example 19) and the used materials and equipment were:

### Materials:

Cells: Primary human umbilical vein cells (HUVEC) were obtained from GIBCO (Cat. C-015-5C).
Cell culture medium: Medium 200 supplemented with LSGS (Gibco, Cat. M-200-500).
Trypsin/EDTA (Gibco, Cat R-001-100)
Trypsin Neutralizer solution (Gibco, Cat R-002-100).
Crystal violet (Merck / Cat. no. 1408)
Ethanol (Riedel-de-Haen / Cat. no. 32205)
Acetic acid (Riedel-de-Haen / Cat. no. 33209)
Culture flasks: Coming, 430168
96 well plates: Greiner, 655 101

### Equipment:

Microtiter pipettes: •Brand Transferpette-8, • Gilson Pipetman 20 and 200 Laminar air flow cabinet: Gelman, Celaire
ELISA reader: Thermo, Multiskan Ascent
Microscope: Leitz Diavert
Balance: Sartorius RC 210 D
CO₂ incubator: Heraeus CO₂-Auto-Zero
Centrifuge: Heraeus Biofuge primo
Cell counter: Assistent, Neubauer hematocytometer

### Example 2

### Pent-4-enoic acid(methoxy-methyl-amide) (9)

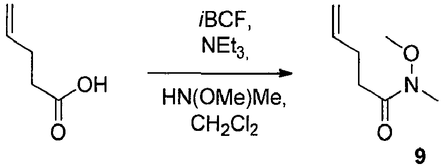

To a solution of 22.0 ml (200 mmol) 4-pentenoic acid and 55.0 ml (400 mmol, 2 eq.) triethylamine in 1.0 L of H₂CCl₂ at 0° C 30.0 ml (220 mmol, 1.10 eq.) *iso*butylchloroformate were added over 30 min. After 10 min of stirring 20.5 g (210 mmol, 1.05 eq.) N,O-diethyl-hydroxylamine and another 55.0 ml triethylamine were added. The mixture immediately started to form bubbles and was stirred over night at RT. The reaction mixture was treated with sat. NaHCO₃ solution (400 ml) and after separation of phases the organic layer was washed with brine (3x 200 ml) and water (1x 200 ml). The solvent was dried over anh. magnesium sulphate and removed *in vacuo.* The crude product was purified via flash chromatography using 30% EtOAc in petroleum ether to afford 27.5 g of a colourless liquid. 15 g of that were distilled in high vacuum to get 14.2 g (corresponding overall yield: 91%) of the desired Weinreb amide
The crude product can be purified by only distilling it; this gives the product in 95 % yield but with a residual content of 1.3 % *iso*butanole (calcd. via NMR).
Boiling point (0.15 mbar): 61° C
R*_{f}*: 0.29 (30% EtOAc in petroleum ether)
Spectral data fit the ones given in literature.

### Example 3

### 1-iodo-hept-6-en-1-yn-3-one (10)

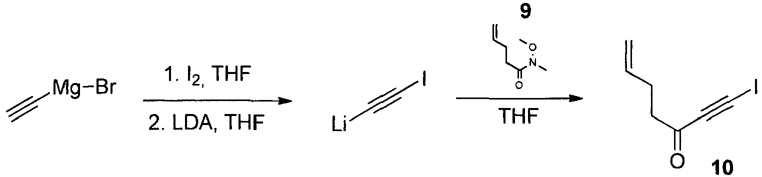

100 ml of an HCCMgBr solution (0.5 M in THF, 50 mmol) were placed in a flask and cooled to -78° C and a solution of 12.6 g iodine (50.0 mmol, 1 eq.) in 38.0 ml THF at - 78° C was added drop wise via cannula. This was stirred for 30 min and then stirred at 0° C for 20 m
in, whereupon the colour of the iodine vanished completely. In parallel a solution of 7.02 ml (50.2 mmol, 1.005 eq.) diisopropylamine in 37.0 ml THF was cooled to -78° C and treated drop wise with 20 ml butyl lithium solution (2.5 M in hexanes, 50.0 mmol, 1 eq.) and stirred for 5 min at -78° C, then warmed to 0° C and stirred for 15 min. Both solutions were cooled to -78° C again and the lithiumdiisopropylamide solution cannulated drop wise to the iodoacetylene sol. This was first stirred for 10 min at -78° C then warmed to 0° C and stirred for further 20 min whereupon the mixture was recooled to - 78°C and 7.16 g (50.0 mmol, 1 eq.) Weinreb-amide **9** added over 30 min, followed by stirring for 10 min., warming to -20°C and stirring for another 2 h until complete conversion due to TLC.

The reaction was quenched by transferring it via cannula into a vigorously stirred emulsion of H₂CCl₂ (300 ml) in a sat. NH₄Cl-soln. (500 ml). After separation of phases the aqueous layer was extracted with H₂CCl₂ (2x 200 ml) and the combined organic layers washed with water (1x 200 ml), dried (MgSO₄) and the solvent was removed *in vacuo.* The crude product was purified via flash chromatography using 10% EtOAc in petroleum ether to elute. This gave 5.03 g (43 %) of an orange liquid which darkens quickly upon irradiation of light and decomposes rather quickly under basic conditions and which could not entirely be separated from the non iodinated by-product.

This reaction can also be performed on scales up to 100 mmol, but the yield dropped to 32%.
R*_{f}*: 0.50 (10% EtOAc in petroleum ether)
¹H-NMR (400 MHz): δ 5.78 (tdd, *J* = 6.4 Hz, 10.4 Hz, 17 Hz, 1 H), 5.03 (m, 2 H), 2.66 (t, *J* = 7.5 Hz, 2 H), 2.42 (td, *J* = 7.5 Hz, 6.4 Hz, 2 H) ppm;
¹³C-NMR (100 MHz): δ 185.5, 136.0, 116.0, 95.0, 44.3, 27.8, 18.3 ppm;
HRMS (ESI) m/z: (M+Na)⁺ calcd for C₇H₇INaO: 256.9434; found: 256.9434;
IR (film): νₘₐₓ 3080, 2980, 2919, 2147, 1669 cm⁻¹

### Example 4

### General triazole synthesis with in-situ generated azides

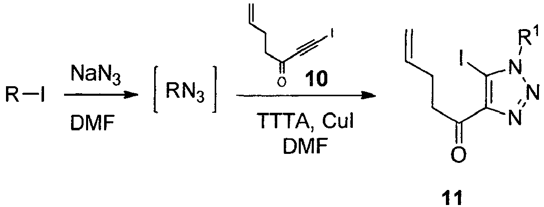

To a suspension of NaN₃ (1 equiv.) in DMF (0.22 M) was added the alkyl iodide (1.5 equiv.). This mixture was stirred in the dark overnight. In a separate flask, CuI (5 mol%) and TTTA (5 mol%) were dissolved in DMF (0.011 M) by stirring the suspension for 40 min to obtain a clear solution. To the catalyst-solution was added iodoalkyne **XX** (1 equiv.) and the resulting mixture was added to the *in situ* generated alkyl azide via cannula. After the reaction was complete (monitored by TLC), it was quenched with 10% NH₄OH in sat. NH₄Cl and diluted with CH₂Cl₂. The layers were separated and the organic phase was washed with 1 M HCl (2x) and water (1x). The combined aqueous layers were the re-extracted with CH₂Cl₂ (2x). The solvent was dried (MgSO₄) and evaporated *in vacuo.* The products were purified as needed by column chromatography.

The following substances were made according to the above described procedure:

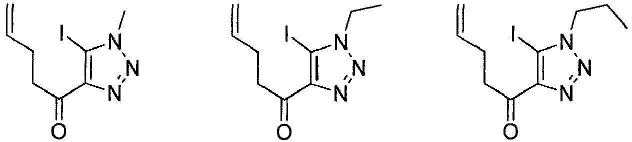

### Example 5

### Triazole synthesis with isolated azides:

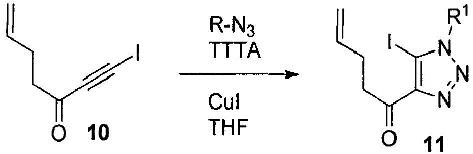

A mixture of CuI (5 mol%) and TTTA (5 mol %) was dissolved in THF (0.011 M) by stirring for 40 min at RT at which time a solution of iodoalkyne **10** (1 eq.) and the azide (1 eq.) in THF (2.0 M) was added. The reaction mixture was monitored by TLC and quenched by the addition of 10% NH₄OH in saturated NH₄Cl. The aqueous layer was then extracted with CH₂Cl₂ (3x). The combined organic layers were dried (MgSO₄) and the solvent evaporated. The crude product was then purified as needed by column chromatography.

The following substances were made according to the above described procedure:

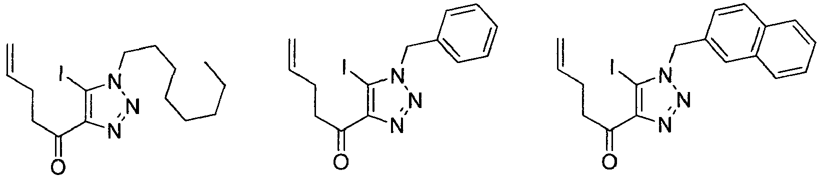

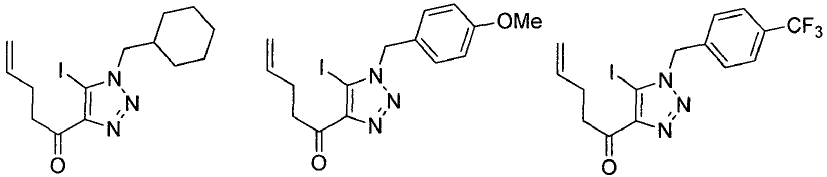

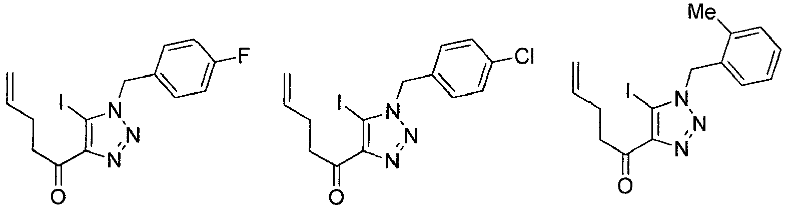

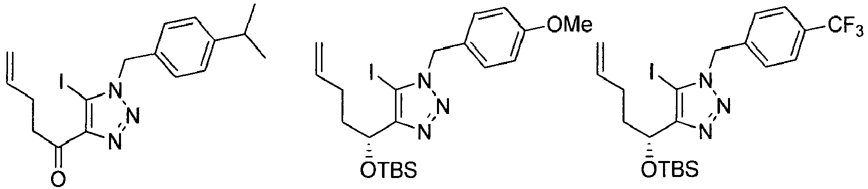

### Example 6

### 1-(5-iodo-1H-imidazol-4-yl)pent-4-en-1-ol (28)

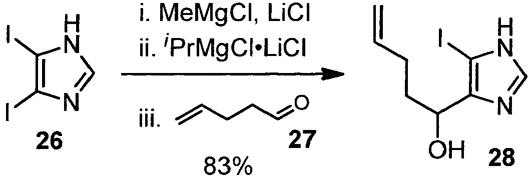

Diiodoimidazole (**26**) (10.06 g, 31.45 mmol) was dissolved in a solution of LiCl (0.5 M in THF, 62.5 mL, 31.25 mmol) in anhydrous THF (105 mL) and cooled to -20 °C under argon whereby a solid precipitated out of solution. Methylmagnesium chloride (10.5 mL, 3.0 M in THF, 31.5 mmol) was added dropwise to make a solution. After 5 min, a solution of isopropylmagnesium chloride•lithium chloride (27.0 mL, 1.3 M in THF, 35.1 mmol) was added dropwise. After the addition was complete, the reaction mixture was warmed to RT. A precipitate formed. The iodine/magnesium exchange was monitored by TLC (mini quench with MeOH) and was complete within 80 minutes. Cooled to -20 °C and aldehyde **27** was added dropwise, neat. The reaction mixture was allowed to slowly warm in the cooling bath and was complete after 2 h. Quenched with half saturated NH₄Cl (300 mL) and the two layers were separated. The aqueous layer was extracted with EtOAc (2 x 100 mL). The combined organics were dried (MgSO₄), filtered, and concentrated on the rotovap until a precipitate began to appear. The solution was then heated to reflux to make a solution with small additions of EtOAc. Hexane added to ring the solution to saturation point. Cooled to RT. Crystals formed. Placed in the fridge overnight. The crystals were collected, washed with 1:1 EtOAc:hexane, then hexane to give 6.80 g (78% yield) of alcohol **28**.

### Example 7

### 1-(5-iodo-1H-imidazol-4-yl)pent-4-en-1-one (29)

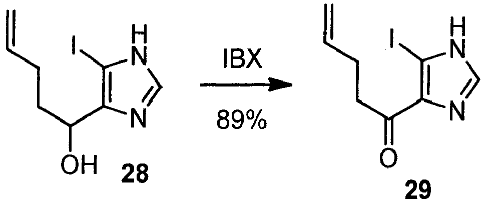

To a solution of alcohol **29** (10.455 g, 37.596 mmol) in DMSO (125 mL) was added IBX (13.687 g, 48.879 mmol) at RT. After 24 h, EtOAc (600 mL) and H₂O (1 L) were added. The mixture was filtered through celite and the layers were separated. The organic phase was washed with H₂O (100 mL) and half-saturated brine (100 mL), dried (MgSO₄), filtered, and concentrated to give 9.252 g (89%) of ketone **29** as a white solid.

### Example 8

### General procedure for the alkylation of ketone 29.

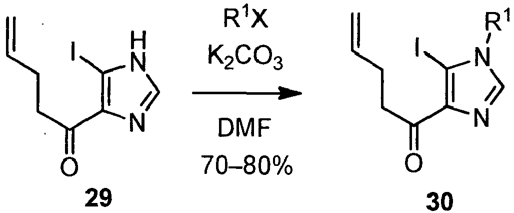

To a solution of ketone **29**, and an alkyl iodide (1.3 equiv) (or an alkyl bromide (1.3 equiv) and tetra-n-butylammonium iodide (0.1 equiv)) in DMF (0.1 M) was added K₂CO₃ (1.5 equiv) and the reaction mixture was stirred under argon until the starting material had been consumed. For hindered electrophiles, heating up to 100 °C was necessary. After completion, the reaction mixture was diluted with EtOAc and H₂O. The two layers were separated and the organic phase was washed with H₂O (twice) and brine (once), then dried (MgSO₄), filtered, and concentrated. The product could be purified by crystallization (EtOAc or CH₂Cl₂ and hexane) or chromatography.
The following substances were synthesized using this technique:

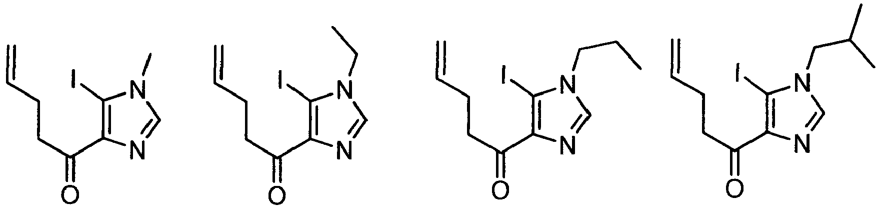

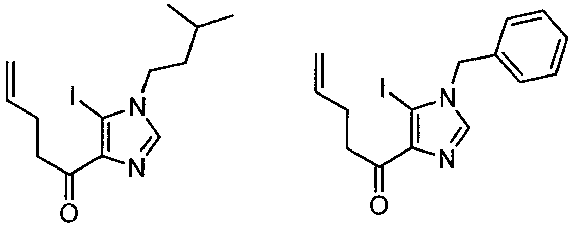

### Example 9

### General Heck cyclization procedure

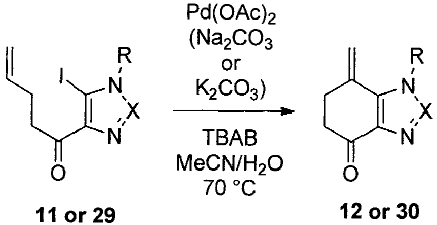

The iodotriazole (or iodoimidazole) **11** or **29** (1 equiv.), TBAB (1 equiv.), Na₂CO₃ (for triazoles) or K₂CO₃ (for imidazoles) (2.5 equiv) and Pd(OAc)₂ (10 mol%) were placed in a Schlenck-tube or flask. The flask was evacuated and flushed with argon (3x). Then a 9:1 MeCN/H₂O mixture (degassed by sparging with nitrogen gas for 30 minutes on an ultrasound bath) was added to bring the concentration to 0.1 M in iodotriazole. The reaction mixture was then stirred in a preheated oil bath at 70° C for 24-72 h. The reaction mixture was diluted with H₂O and EtOAc, filtered through celite and the two layers were separated. The aqueous layer was extracted with EtOAc (2x), and the combined organics were dried (MgSO₄), filtered, and concentrated. The product was purified by column chromatography using mixtures of EtOAc and petroleum ether.

The following substances were synthesized in this manner:

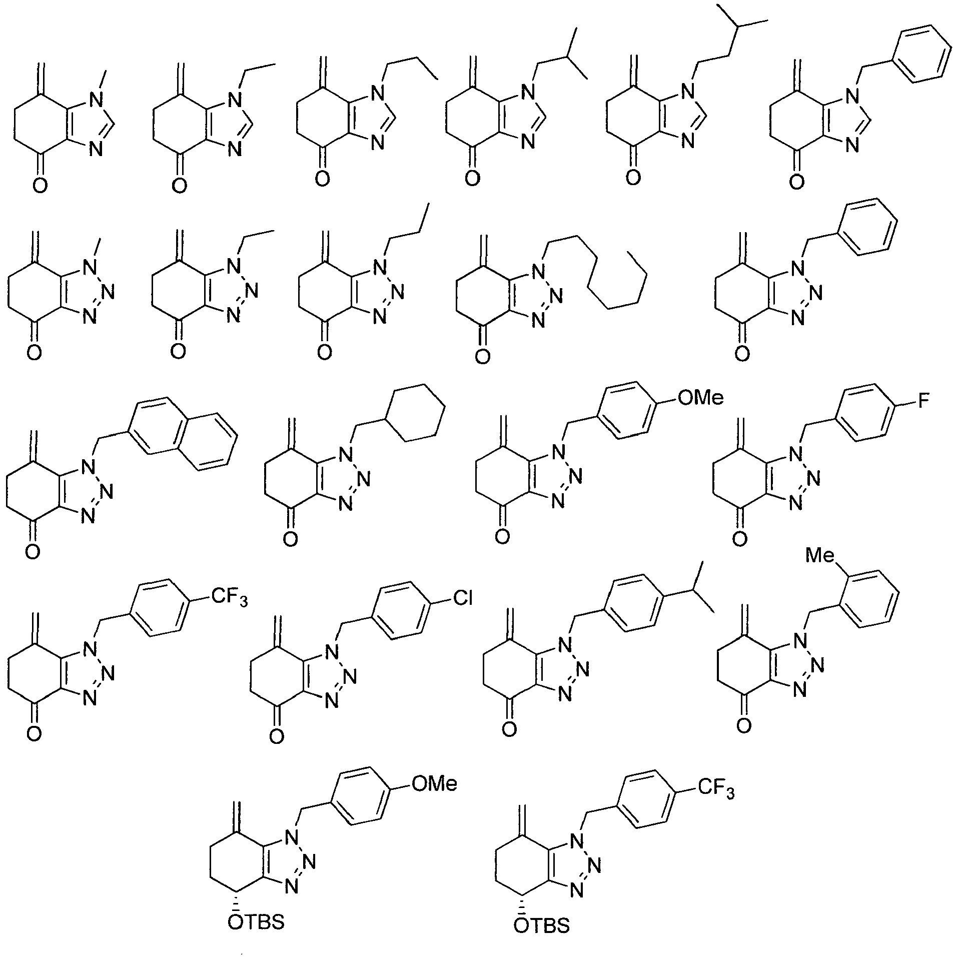

### Example 10

### General DIBAL reduction:

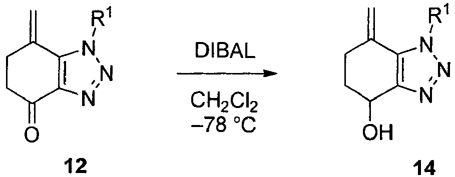

To a solution of ketone **12** in CH₂Cl₂ (0.1 M) was added a solution of DIBAL (1.5 equiv) at -78 °C. After 40 min, the reaction was quenched with a few drops of MeOH and then with 20% Rochelle's salts, diluted with CH₂Cl₂, and stirred vigorously for 1.5 h. The mixture was filtered through celite and the two layers were separated. The aqueous layer was extracted with CH₂Cl₂ (twice), and the combined organics were dried (MgSO₄), filtered, and concentrated. The product was purified by chromatography.
The following substances were synthesized in this manner:

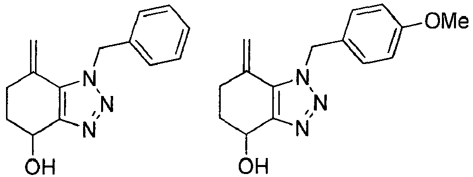

### Example 11

### General esterification reaction:

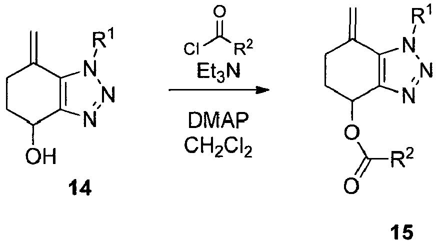

To a solution of alcohol **14** and DMAP (10 mol%) in CH₂Cl₂ (0.3 M) was added the acid chloride (1.2 equiv) and triethylamine (1.4 equiv) at RT. After 18 h, the reaction mixture was diluted with saturated NaHCO₃ and diluted with CH₂Cl₂. The two layers were separated and the aqueous layer was extracted with CH₂Cl₂ (3 times). The combined organics were washed with saturated NaHCO₃ (twice), dried (MgSO₄), filtered, and concentrated. The product was purified by chromatography.
The following substances were made using this method:

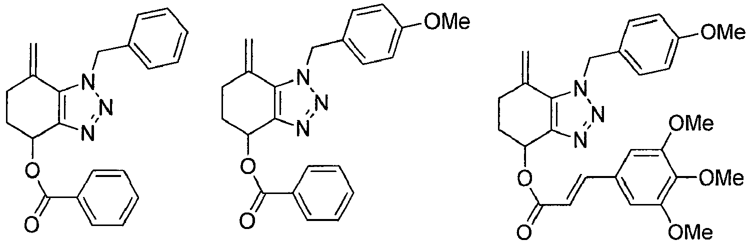

### Example 12

### Synthesis of carbamates 19 from alcohol 14:

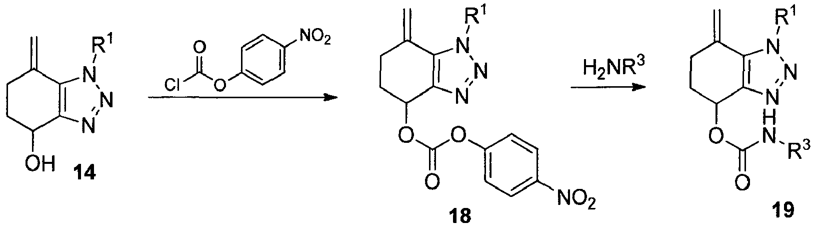

To a solution of alcohol **14** and DMAP (0.2 equiv) in CH₂Cl₂ (0.1 M) was added triethylamine (10 equiv) and a solution of 4-nitrophenyl chloroformate (2.5 equiv) in CH₂Cl₂ (0.8 M) at 0 °C. After addition, the reaction was allowed to warm to RT. After 1.5 h, the reaction was quenched with H₂O (20 mL) and diluted with CH₂Cl₂ (20 mL). The two layers were separated and the aqueous layer was extracted with CH₂Cl₂ (2 x 10 mL). The combined organics were dried (MgSO₄), filtered, and concentrated. The product was purified by chromatography to give **18** as a yellow solid. To a suspension of carbonate **18** a primary amine (5 equiv) in EtOH (0.05 M) was added iPr₂NEt (5 equiv) at RT. After 40 min, the suspension had clarified to a yellow solution, and after 2 h, the reaction was diluted with H₂O (80 mL) and extracted with EtOAc (3 x 30 mL). The combined organics were dried (MgSO₄), filtered, and concentrated. The product was purified by chromatography.

The following substances were synthesized by this method:

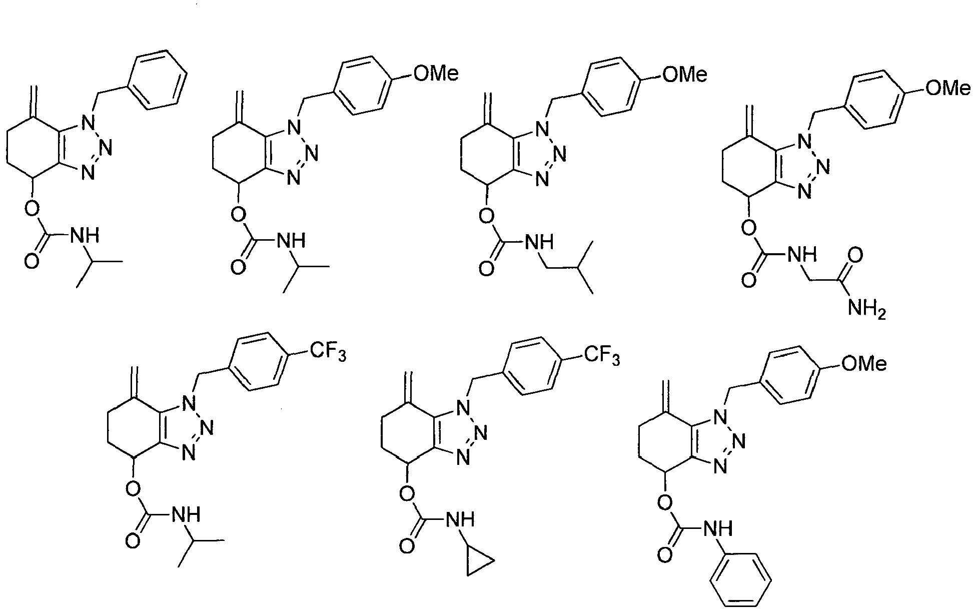

### Example 13

### Reductive amination of ketone 12

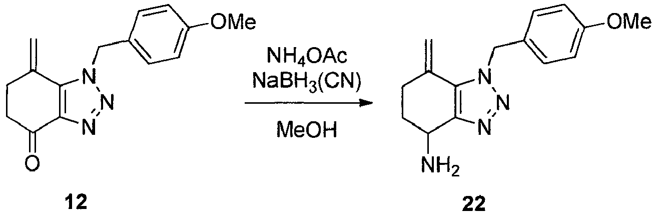

**1-(4-methoxybenzyl)-7-methylene-4,5,6,7-tetrahydro-1H-benzo[d] [1,2,3]triazol-4-amine (22):** To a solution of ketone **12** (504 mg, 1.87 mmol) and NH₄OAc (1.91 g, 24.8 mmol) in MeOH (20 mL) was added NaBH₃(CN) (593 mg, 9.43 mmol) at RT. After 20 h the reaction mixture was loaded directly onto silica gel and purified by column chromatography (5% then 10% MeOH in CH₂Cl₂) to give 263 mg (52%) of **22** as a colorless oil.
**R***_{f}*: 0.45 (5% MeOH in CH₂Cl₂)
¹**H NMR** (400 MHz): δ 7.00-6.94 (m, 2 H), 6.88-6.82 (m, 2 H), 6.13 (s, 2 H), 5.64-5.52 (m, 2 H), 5.24 (s, 1 H), 5.20 (s, 1 H), 4.87-4.79 (m, 1H), 3.75 (s, 3 H), 2.73-2.65 (m, 1 H), 2.57-2.34 (m, 2 H), 2.17-2.09 (m, 1 H) ppm

### Example 14

### Carbamate synthesis from amine 22

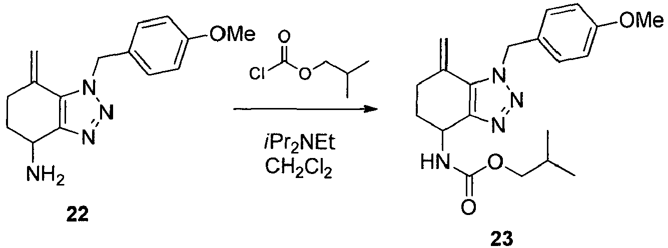

**Isobutyl (1-(4-methoxybenzyl)-7-methylene-4,5,6,7-tetrahydro-1H-benzo[d] [1,2,3]triazol-4-yl)carbamate (23):** To a solution of amine **22** (97 mg, 0.36 mmol) and *i*Pr₂NEt (91.5 µL, 0.538 mmol) in CH₂Cl₂ (2.5 mL) was added isobutyl chloroformate (56 µL, 0.43 mmol) at RT. After 15 min, the reaction mixture was quenched with H₂O (20 mL) and extracted with CH₂Cl₂ (3 x 10 mL). The combined organics were dried (MgSO₄), filtered, and concentrated. The product was purified by MPLC (0-5% gradient of MeOH in CH₂Cl₂) to give 88 mg (66%) of **23** as a white solid.
**R*_{f}***: 0.59 (5% MeOH in CH₂Cl₂)
**¹H NMR** (400 MHz): δ 7.07-7.01 (m, 2 H), 6.89-6.83 (m, 2 H), 5.63 (s, 2 H), 5.24-5.10 (m, 3 H), 5.04-4.97 (m, 1 H), 3.95-3.85 (m, 2 H), 3.79 (s, 3 H), 2.60-2.41 (m, 2 H), 2.31 (bs, 1 H), 2.00-1.76 (m, 2 H), 0.94 (d, *J*= 6.7 Hz, 6 H) ppm

### Example 15

### General Epoxidation Method:

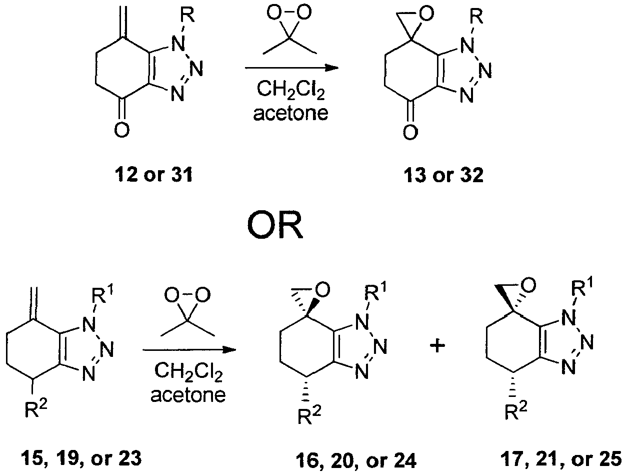

To a solution of alkene in CH₂Cl₂ (0.1 M) was added a solution of DMDO in acetone (~0.09M, 1.5-2.5 equiv.) at RT. The reaction was monitored by TLC and concentrated when complete. The product(s) were purified by chromatography.

**All of the preferred compounds as shown above on page 10 and claim 2 were made following this general epoxidation method.**

### Example 16

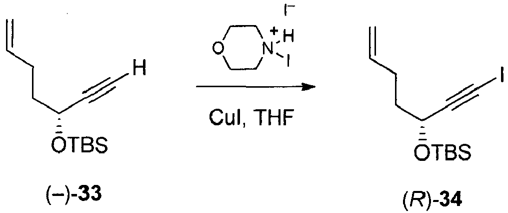

**(*R*)-tert-butyl((1-iodohept-6-en-1-yn-3-yl)oxy)dimethylsilane (34):** To a solution of terminal alkyne (-)-**33** (1.607 g, 7.163 mmol, >95% ee)¹ and freshly prepared NIM (2.933 g, 8.603 mmol) in THF (25 mL) was added CuI (70.0 mg, 0.368 mmol) at RT. After 20 h, the reaction mixture was filtered through a pad of basic alumina and washed with CH₂Cl₂ (4 x 50 mL). The filtrate was concentrated and the product was purified by column chromatography (0-5% EtOAc in petroleum ether) to give 2.384 g (95%) of 34 as a pale yellow liquid. NOTE: Use of freshly prepared NIM was crucial for the success of the reaction. When older batches were used, the reaction could not be driven to completion and the starting material and product were nearly impossible to separate by chromatography.
¹ A. Fürstner, C. Nevado, M. Waser, M. Tremblay, C. Chevrier, F. Teplý, C. Aïssa, E. Moulin, O. Müller, J. Am. Chem. Soc. 2007, 129, 9150-9161.

**¹H-NMR** (400 MHz): δ 5.80 (ddt, *J*= 17.1, 10.2, 6.5 Hz, 1 H), 5.04 (dq, *J*= 17.1, 1.8 Hz, 1 H), 4.98 (m, 1 H), 4.49 (t, *J* = 6.5 Hz, 1 H), 2.22-2.14 (m, 2 H), 1.79-1.73 (m, 2 H), 0.90 (s, 9 H), 0.13 (s, 3 H), 0.10 (s, 3 H) ppm
**HRMS** (ESI) m/z: (M+Na)⁺ calcd for C₁₃H₂₃INaOSi: 373.0455; found: 373.0453

### Example 17

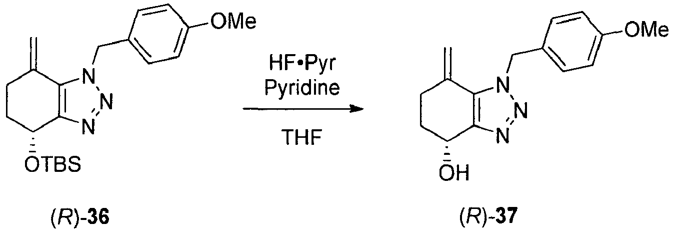

**(R)-1-(4-methoxybenzyl)-7-methylene-4,5,6,7-tetrahydro-1H-benzo[d] [1,2,3] triazol-4-ol (37):** To a 0 °C solution of silyl ether (*R*)-**36** (249 mg, 0.646 mmol) in THF (13 mL) was added pyridine (766 µL, 9.47 mmol) followed by HF•pyridine (1.133 mL). After 3 h, the reaction was carefully quenched with sat'd K₂CO₃ solution (21 mL). The mixture was then extracted with EtOAc (3 x 30 mL). The combined organics were dried (MgSO₄), filtered, and concentrated to give 171 mg (97%) of crude (*R*)-**37** which was sufficiently pure by ¹H NMR to be used in the next step. The following substance was synthesized in the same manner:

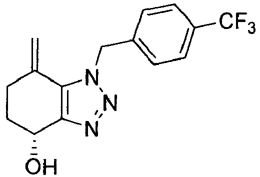

### Example 18

### Measurement of the MetAP inhibitory activity of the compounds of the present invention

The below protocol describes the measurement of the inhibitory activity of the compounds of the present invention by using *E*. *coli* MetAP, hsMetAP-I, and hsMetAP-II.

Inhibition activity of compounds was studied using the tetrapeptide substrate Met-Gly-Met-Met (Bachem) followed by HPLC analysis of the product Gly-Met-Met. The assay was performed in 96 well plates (Greiner) at a total reaction volume of 100 µl. First, enzyme (final concentration: 50 nM) was incubated with inhibitor (DMSO solution, final concentration: 25 µM) in reaction buffer (final concentration: 100 µM cobalt(II) chloride hexahydrate, 100 mM sodium chloride, 0.075 % bovine serum albumine, 50 mM Tris pH 7.5) for 20 minutes at 37 °C. A positive control with DMSO solution instead of inhibitor at the corresponding concentration was prepared. Then the substrate Met-Gly-Met-Met was added to a final concentration of 400 µM. After an incubation period of 15 minutes (HsMetAP-I), 20 minutes (EcMetAP) or 60 minutes (HsMetAP-II) at 37 °C, the enzyme reaction was stopped by adding 10 µl 4 % trifluoroacetic acid. The plate was centrifuged at 1000 x g for 10 minutes.

Detection of the product Gly-Met-Met was performed by HPLC (Jasco), and typically using the following, or equivalent conditions: C18 column (Dr. Maisch, ReproSil-Pur® 120 ODS-3, 3 µm, 50 x 2 mm). 80 µl of sample were applied on the column and eluted with mobile phase A (water and 0.1 % trifluoroacetic acid, filtered, degassed) and B (acetonitrile and 0.1 % trifluoroacetic acid, degassed) over 8 minutes. The gradient started with 10 % mobile phase B, rising linearly to 58 % B over 4 minutes for elution of enzyme reaction product, followed by 95 % B for 2 minutes to remove any residual material off the column before re-equilibration at initial conditions for 2 minutes. The product was quantified by UV absorption at 214 nm

Percentage inhibition was calculated as the relation of Gly-Met-Met amount resulting in inhibited and uninhibited enzyme reaction (positive control). IC50 values were determined by measuring the inhibition at a wide range of inhibitor concentrations (varying from near-complete inhibition to no effect). The values were determined in relation to positive controls with equal DMSO contents. The inhibition values for the varying concentrations of inhibitor were then fitted to a logarithmic dose-response curve and the IC50 values were calculated from the equation thus obtained.

The results are shown in Table 1.

### Example 19

### Measurement of the HUVEC growth arrest IC₅₀ values

### Preparation of Stock Solutions

Crystal violet solution:
Crystal violet (Merck) was dissolved in ethanol (PA quality, Roth) to a concentration of 10 %. The solution was diluted with ddH₂O 1:20.

Test compounds:
Compounds of the present invention were dissolved at a concentration of 10 mM in DMSO (Roth, Cat. 47201). Stock solutions of test compounds were diluted 1:10 not earlier than 1 hour before the assay was made.

### Assay method:

1. HUVEC cells were seeded in 96-well plates (300 cells/plate).
2. 6 hours later test compounds were added in triplicates Concentrations of test compound: 0, 1.6, 3.2, 6.3, 12.5, 25, 50, 100 µM. Fresh medium and test compounds were added every 48 hr.
3. One 96 well plate (t=0) with untreated cells was fixed at the same time point as treatment of cells with test compounds was initiated.
4. Test plates were incubated for 10 days in a CO₂ incubator.
5. Fixation of cells: Wells were washed with 100 µL/well PBS. PBS was decanted and 100 µL of 3 % formaldehyde/well was added. After 5 min, formaldehyde was decanted and plates were dried upside down on blotting paper overnight at room temperature.
6. Staining: 0.1 mL of crystal violet solution was added to each well with a 12-channel pipette. After incubation for 5 min at room temperature, the crystal violet solution was decanted and plates were washed 5 x by submersion in 3 L fresh water.
7. After drying (plates were dried on blotting paper upside down overnight) 0.1 mL of ethanol/acetic acid (99:1) was added to each well and the optical density at 595 nm was determined in an ELISA reader.

The evaluation of the growth inhibitory activity was carried out as follows:
Definitions: Growth: Mean OD at t=10 d - mean OD at t=0.
Evaluation: Dose response curves were established by plotting growth against concentration of compounds using Microsoft Excel. The IC₅₀ was determined from the dose response curves by computerized calculation of crossing points of dose response curves with a line parallel to the x-axis corresponding to 50% of uninhibited growth.

The results of the above described experiments are shown in Table 1.

Thus, the compounds HHS-I-028-B, BK-I-161-A, UWU-XII-023-A, UWU-XII-035-A, UWU-XII-128-A, and (+)-UWU-XII-131-A show a good inhibitory effect on MetAP-II and are thus, useful as anti-angiogenic compounds.

**Table 1**

| Structure | Code | E. Coli % inhibition | hsMetAP-I % inhibition | hsMetAP-II % inhibition | hsMetAP-II IC₅₀ (µM) | HUVEC IC₅₀ (µM) |
|---|---|---|---|---|---|---|
| 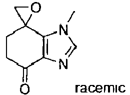 | JL-II-136-A | Inactive | Inactive | Inactive | NA | NA |
| 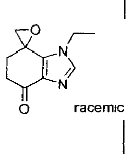 | JL-II-137-A | Inactive | Inactive | Inactive | NA | NA |
| 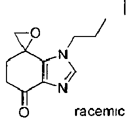 | JL-II-116-A | Inactive | Inactive | Inactive | NA | NA |
| 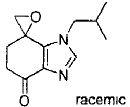 | JL-II-120-B | Inactive | Inactive | 12±6 | NA | NA |
| 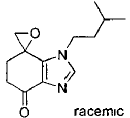 | JL-II-115-A | 25 ± 8 | Inactive | Inactive | NA | NA |
| 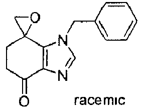 | JL-III-084-C | Inactive | Inactive | 24 ± 1 | NA | NA |
| 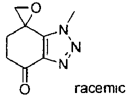 | CJ-II-011 | 20 ± 2 | 14 ± 7 | Inactive | NA | NA |
| 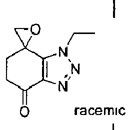 | CJ-II-007 | 22 ± 5 | Inactive | Inactive | NA | NA |
| 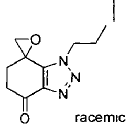 | CJ-II-012 | 21 ± 5 | 13 ± 5 | 25 ± 2 | NA | NA |
| 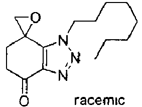 | CJ-II-010 | 14 ± 7 | 12.1 ± 1.5 | 50 ± 2 | NA | NA |
| 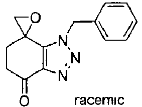 | HHS-I-027-B | Inactive | Inactive | 64.5 ± 0.1 | 13.7 ± 3.4 | NA |
| 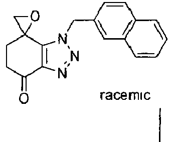 | HHS-I-020-C | 24 ± 8 | Inactive | 84.1 ± 3.2 | 2.7 ± 0.1 | NA |
| 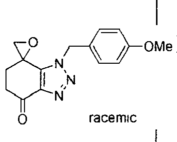 | HHS-I-028-B | Inactive | Inactive | 97.0 ± 0.3 | 0.98 ± 0.03 | 8.8 |
| 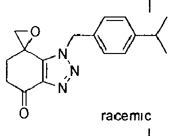 | JL-III-138-A | 50 ± 6 | 16 ± 2 | 84 ± 3 | 5.5 ± 3 | NA |
| 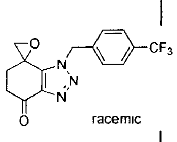 | BK-I-161-A | Inactive | Inactive | 100 | 0.52 ± 0.05 | 0.9 |
| 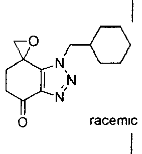 | BK-I-177-A | 19 ± 5 | Inactive | 40 ± 3 | 17 ± 6 | 9.7 |
| 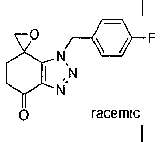 | BK-I-178-A | 13 ± 5 | Inactive | 100 | 0.87 ± 0.09 | 3.2 |
| 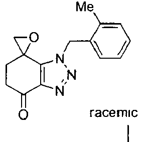 | BK-I-181-B | Inactive | Inactive | 100 | 5.1 ± 2.9 | NA |
| 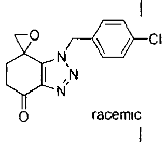 | BK-I-184-A | 14 ± 5 | Inactive | 100 | 0.74 ± 0.03 | 7.4 |
| 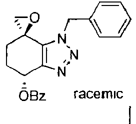 | JL-III-147-A | 45 ± 7 | 15 ± 1 | 93.3 ± 2.1 | 1.3 ± 0.01 | NA |
| 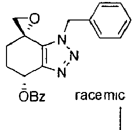 | JL-III-147-B | 36 ± 11 | Inactive | 59 ± 2 | 19.1 ± 9 | NA |
| 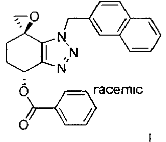 | JL-III-132-C | 22 ± 7 | Inactive | 63 ± 5 | 3.7 ± 0.3 | NA |
| 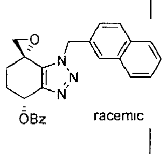 | JL-III-132-D | Inactive | Inactive | 15 ± 5 | NA | NA |
| 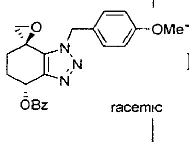 | UWU-II-023-A | Inactive | Inactive | 98.6 ± 0.4 | 0.60 ± 0.01 | 2.4 |
| 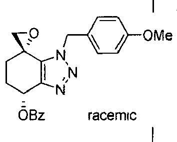 | UWU-XII-023-D | Inactive | Inactive | 45 ± 4 | 17 ± 12 | 2.1 |
| 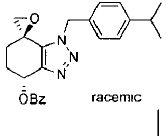 | UWU-XII-036-A | 20 ± 8 | Inactive | 59 ± 5 | 4.7 ± 0.3 | NA |
| 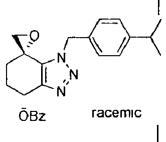 | HHS-I-036-A2 | 22 ± 12 | Inactive | 15 ± 1 | NA | NA |
| 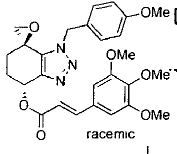 | UWU-XII-035-A | Inactive | Inactive | 94 ± 4 | 0.5 ±1 | NA |
| 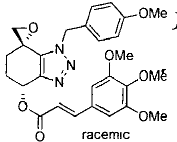 | UWU-II-035-C | Inactive | Inactive | 85 ± 11 | 2.02 ± 0.03 | NA |
| 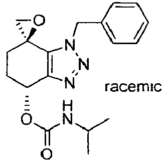 | UWU-XII-128-A | 24 ± 2 | Inactive | 100 | 0.42 ± 0.07 | 1.2 |
| 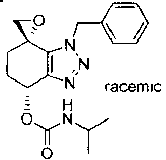 | UWU-XII-128-C | 18 ± 2 | Inactive | 60 ± 5 | 18 ± 9 | 9.5 |
| 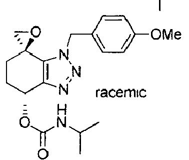 | UWU-XII-131-A | Inactive | Inactive | 100 | 0.14 ± 0.01 | 0.4 |
| 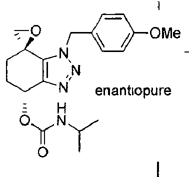 | (+)-UWU-XII-131-A | Inactive | Inactive | 100 | NA | 0.3 |
| 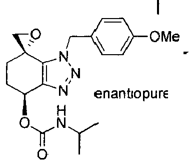 | (-)-UWU-XII-131-A | Inactive | Inactive | 19 ±10 | NA | 8.2 |
| 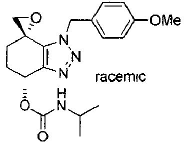 | UWU-XII-131- C | Inactive | Inactive | 76 ± 1 | 4.9 ± 1.0 | 2.5 |
| 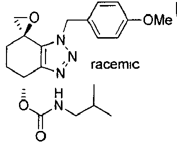 | UWU-XII-142- A | 18.7 ±0.8 | Inactive | 100 | 0.15 ± 0.03 | 2.4 |
| 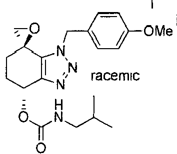 | UWU-XII-142- C | 18 ± 2 | Inactive | 72 ± 1 | 4.0 ±1.3 | 7.1 |
| 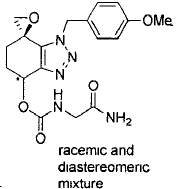 | UWU-XII-143- A | 12 ± 1 | Inactive | 100 | 0.23 ± 0.02 | 2.6 |
| 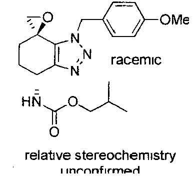 | UWU-XII-144- A | 25 ± 3 | 15 ± 3 | 100 | 0.6 ± 0.1 | 2.5 |
| 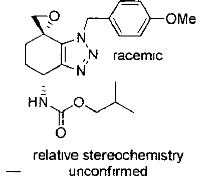 | UWU-XII-144- B | 47 ± 1 | 16 ± 6 | 100 | 0.76 ± 0.03 | 7.8 |
| 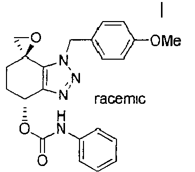 | UWU-XII-149- A | Inactive | Inactive | 100 | 0.99 ± 0.05 | 3.2 |
| 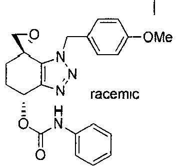 | UWU-XIII- 005-A | Inactive | Inactive | 44 ± 6 | 18 ± 5 | 14.9 |

## Claims

1. A compound of the general formula (I) 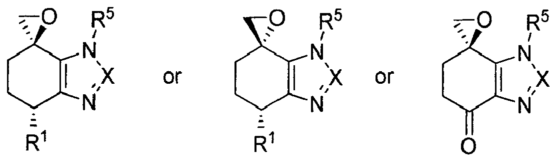 X=CorN
R¹ = OC(O)R² where R² is alkyl, cycloalkyl, aryl, heteroaryl, or cinnamyl or
R¹ = OC(O)NHR³ where R³ is alkyl, cycloalkyl, aryl, or heteroaryl
or
R¹ = NHC(O)OR⁴ where R⁴ is alkyl
or
R¹ = OH, NH₂
R⁵ = alkyl, cycloalkyl, CH₂R⁶ where R⁶ is aryl or heteroaryl

2. The compound of claim 1, wherein the compound is

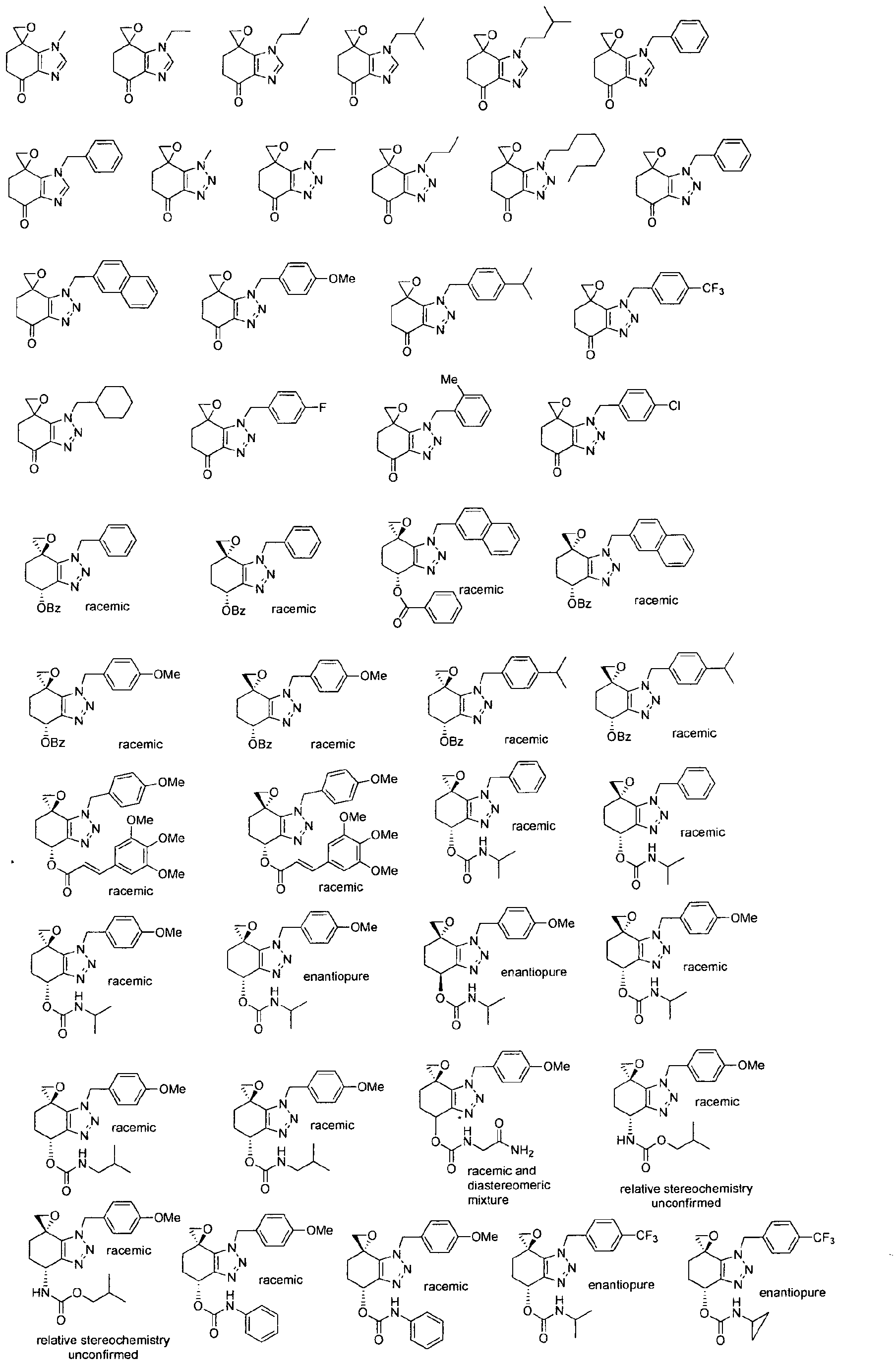

3. The compound of claim 1 or 2, wherein the compound is

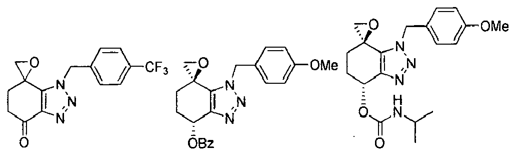

4. A pharmaceutical composition comprising at least one compound of claim 1, 2 or 3 together with pharmaceutically acceptable carriers and/or excipients.

5. The pharmaceutical composition of claim 4 comprising further active agents selected from the group consisting of nucleoside analogs, alkylating agents, corticosteroids, angiogenesis inhibitors, interferon and anti-cancer antibodies.

6. A compound of the general formula (I) 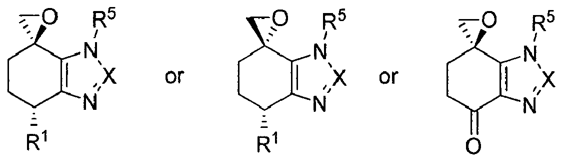 X=CorN
R¹ = OC(O)R² where R² is alkyl, cycloalkyl, aryl, heteroaryl, or cinnamyl or
R¹ = OC(O)NHR³ where R³ is alkyl, cycloalkyl, aryl, or heteroaryl
or
R¹ = NHC(O)OR⁴ where R⁴ is alkyl
or
R¹ = OH, NH₂
R⁵ = alkyl, cycloalkyl, CH₂R⁶ where R⁶ is aryl or heteroaryl
for use in a method of inhibiting angiogenesis.

7. The compound for the use of claim 6, wherein the angiogenesis is inhibited for treating cancer, ophthalmic diseases, skin diseases, rheumatoid arthritis and obesity.

8. The compound for the use of claim 7, wherein the cancer is melanoma, basal cells carcinoma, Kaposi sarcoma, glioblastoma, neuroblastoma, cancer of the lung, bladder, prostate, kidney, testis, intestine, colon, spleen, cervix, ovary, endometrium, uterus, esophagus, tongue, bronchi, pancreas, liver, breast, stomach or bone, leukemia, lymphoma or myeloma.

9. A method for preparing a compound of Formula (I) wherein X = N as defined in any of claims 1 to 3, comprising the following steps: 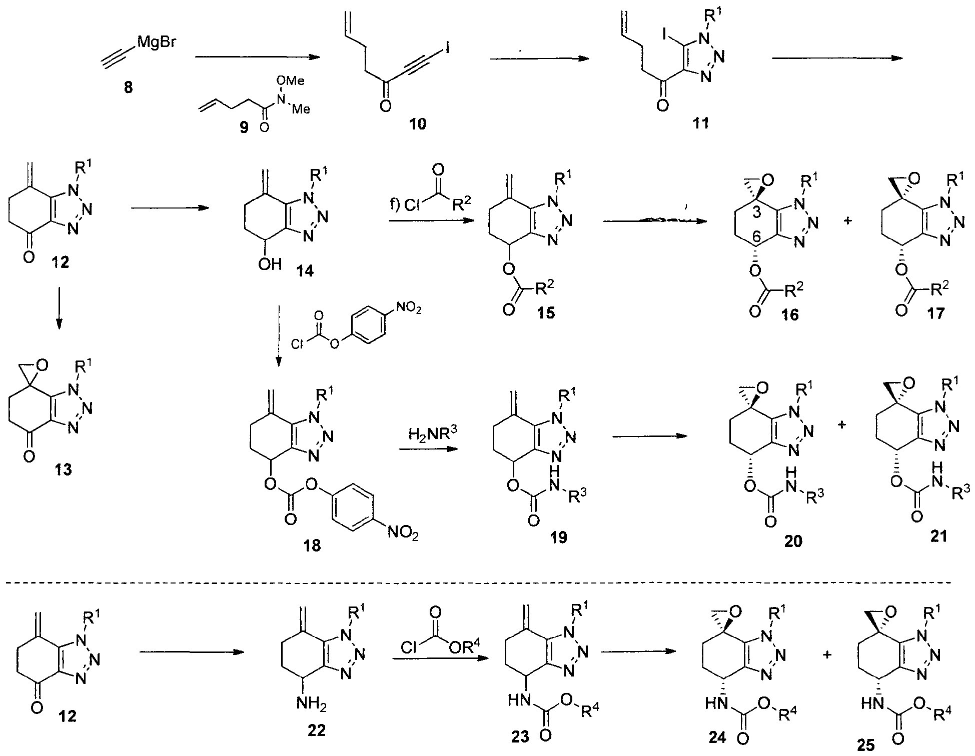
(a) iodination and subsequent deprotonation of ethynylmagnesium bromide (**8**) to give lithium iodoacetylide which reacts with Weinreb amide **9** to give iodoynone **10**,
(b) reaction of Alkyne **10** with an azide in a Huisgen cyclizations to generate triazoles **11**,
(c) providing bicyclic olefins **12** by using Jeffery's phase transfer method,
(d) Epoxidation of **12** to provide **13**; or
esterification of **12** to **15**, via alcohol **14** followed by epoxidation to give a 1:1 diastereomeric mixture of epoxides **16** and **17**; or
conversion of **12** to carbamates **19**, via alcohol **14** and carbonate **18**, followed by epoxidation to a 1:1 diastereomeric mixture of **20** and **21**; or
reductive amination of **12** to give amines **22**, which are convertable into carbamates **23** and epoxides **24** and **25.**

10. A method for preparing a compound of Formula (1) wherein X = C as defined in any of claims 1 to 3, comprising the following steps: 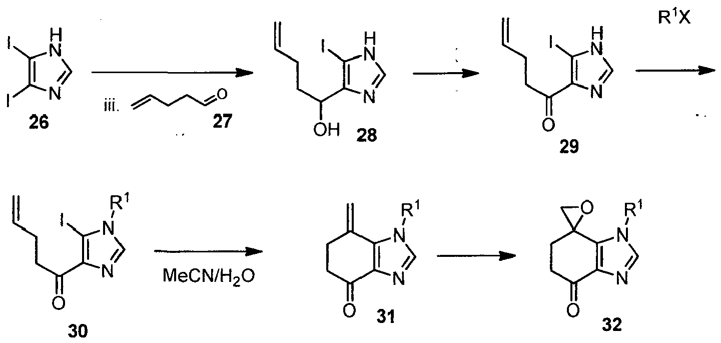
(a) Magnesium/halogen exchange on diiodoimidazole **(26)** using Knochel's procedure, followed by addition of aldehyde **27** to give alcohol **28** as a crystalline racemic solid,
(b) Oxidation of the alcohol **28** to provide ketones **29**
(c) Alkylation of ketones **29** with an electrophile to give imidazoles **30**,
(d) Heck cyclization imidazoles **30** to provide the bicyclic olefins **31**,
(e) Epoxidation of the olefins **31** to the final products **32**.
